# EUROPÄISCHE PATENTSCHRIFT

(11) **EP 0 830 863 B1**
(45) Veröffentlichungstag und Bekanntmachung des Hinweises auf die Patenterteilung: **10.05.2000**
(21) Anmeldenummer: 97115603.9
(22) Anmeldetag: 09.09.1997
(51) Int. Cl.: A61K 31/55

(54) **Die gastrointestinale Durchblutung fördernde Arzneimittel**
Drugs for increasing gastrointestinal blood supply
Médicaments améliorateurs de l'irrigation sanguine gastrointestinale

(30) Priorität: 18.09.1996 DE 19638020
(43) Veröffentlichungstag der Anmeldung: 25.03.1998
(73) Patentinhaber: Solvay Pharmaceuticals GmbH, 30173 Hannover (DE)
(72) Erfinder: Rozsa, Susanna, Dr., 6722 Szeged / Ungarn (HU); Gy. Papp, Julius, Prof. Dr., 6722 Szeged / Ungarn (HU); Thormählen, Dirk, Dr., 31039 Rheden (DE); Waldeck, Harald, Dr., 30916 Isernhagen (DE)
(74) Vertreter: Lauer, Dieter, Dr.

(56) Entgegenhaltungen:
- EP-A- 0 733 642
- WO-A-94/26719

## Beschreibung

Die vorliegende Erfindung betrifft die Verwendung von Benzazepin-N-essigsäurederivaten, welche in α-Stellung zu dem Stickstoffatom eine Oxogruppe enthalten und in 3-Stellung durch einen 1-(Carboxyalkyl)-cyclopentyl-carbonyl-amino-Rest substituiert sind, und deren Salzen und biolabilen Estern zur Behandlung und/oder Prophylaxe von Durchblutungsstörungen im Magendarmbereich und zur Herstellung von für diese Behandlung geeigneten Arzneimitteln.

Der Erfindung liegt die Aufgabe zugrunde, neue pharmazeutische Zubereitungen zur Verbesserung der Durchblutung im Magendarmbereich und zur Behandlung und/oder Prophylaxe von im Zusammenhang mit Durchblutungsstörungen im Magendarmbereich auftretenden Symptomen zu entwickeln.

Erfindungsgemäß werden zur Herstellung von pharmazeutischen Zubereitungen zur Behandlung und/oder Prophylaxe von Durchblutungsstörungen im Magendarmbereich Verbindungen der allgemeinen Formel I 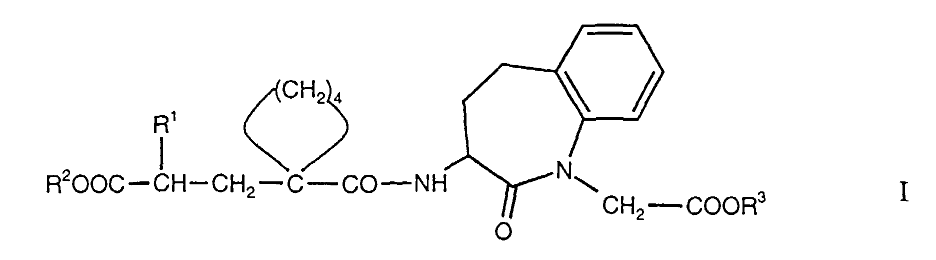 worin
- R¹: für eine Phenyl-C1-C4-alkylgruppe, welche gegebenenfalls im Phenylring durch C1-C4-Alkyl, C1-C4-Alkoxy oder Halogen substituiert sein kann, oder für eine Naphthyl-C1-C4-alkylgruppe steht,
- R²: Wasserstoff oder eine einen biolabilen Ester bildende Gruppe bedeutet und
- R³: Wasserstoff oder eine einen biolabilen Ester bildende Gruppe bedeutet
und physiologisch verträgliche Salze der Säuren der Formel I verwendet.

Sofern in den Verbindungen der Formel I die Substituenten C1-C4-Alkyl- oder Alkoxygruppen bedeuten oder enthalten, können diese geradkettig oder verzweigt sein und insbesondere 1 bis 4, vorzugsweise 1 bis 2, Kohlenstoffatome enthalten und stellen bevorzugt Methyl oder Methoxy dar. Sofern die Substituenten Halogen enthalten, kommen insbesondere Fluor, Chlor oder Brom, vorzugsweise Fluor oder Chlor in Frage.

In dem Rest R¹ kann C1-C4-alkylenkette 1 bis 4, vorzugsweise 1 bis 2 Kohlenstoffatome enthalten. Insbesondere stellt R¹ eine gegebenenfalls substituierte Phenethylgruppe dar, welche gegebenenfalls ein- oder mehrfach durch Halogen, C1-C4-Alkoxy oder C1-C4-Alkyl substituiert sein kann, oder eine Naphthylethylgruppe.

Die Verbindungen der Formel I stellen gegebenenfalls veresterte Dicarbonsäurederivate dar. Je nach Applikationsform sind biolabile Monoester, insbesondere Verbindungen, worin R² eine einen biolabilen Ester bildende Gruppe und R³ Wasserstoff bedeuten, oder Dicarbonsäuren bevorzugt, wobei letztere insbesondere für i.v.-Applikation geeignet sind.

Als biolabile Ester bildende Gruppen R² und R³ eignen sich C1-C4-Alkylgruppen, gegebenenfalls im Phenylring durch C1-C4-Alkyl oder durch eine an zwei benachbarte Kohlenstoffatome gebundene C1-C4-Alkylenkette substituierte Phenyl- oder Phenyl-C1-C4-alkylgruppen, im Dioxolanring gegebenenfalls durch C1-C4-Alkyl substituierte Dioxolanylmethylgruppen oder gegebenenfalls an der Oxymethylgruppe durch C1-C4-Alkyl substituierte C₂-C₆-Alkanoyloxymethylgruppen. Sofern die einen biolabilen Ester bildende Gruppe R² oder R³ C1-C4-Alkyl bedeutet, kann dieses eine bevorzugt unverzweigte Alkylgruppe mit 1 bis 4, vorzugsweise 2 Kohlenstoffatomen darstellen. Sofern die einen biolabilen Ester bildende Gruppe eine gegebenenfalls substituierte Phenyl-C1-C4-alkylgruppe darstellt, kann deren Alkylenkette 1 bis 3, vorzugsweise 1, Kohlenstoffatome enthalten. Sofern der Phenylring durch eine C1-C4-Alkylenkette substituiert ist, kann diese 3 bis 4, insbesondere 3 Kohlenstoffatome enthalten. Als phenylhaltige Substituenten R² und/oder R³ eignen sich insbesondere Phenyl, Benzyl oder Indanyl. Sofern R² und/oder R³ eine gegebenenfalls substituierte Alkanoyloxymethylgruppe darstellen, kann deren Alkanoyloxygruppe 2 bis 6, vorzugsweise 3 bis 5, Kohlenstoffatome enthalten und ist vorzugsweise verzweigt und kann beispielsweise einen Pivaloyloxymethylrest (= tert.-Butylcarbonyloxymethylrest) darstellen.

Als physiologisch verträgliche Salze von Dicarbonsäuren oder Monoestern der Formel I kommen deren Alkalimetall-, Erdalkalimetall- oder Ammoniumsalze in Frage, beispielsweise Natrium- oder Kalziumsalze oder Salze mit physiologisch verträglichen, pharmakologisch neutralen organischen Aminen wie beispielsweise Diethylamin oder tert.-Butylamin.

Die Verbindungen der Formel I enthalten zwei chirale Kohlenstoffatome, nämlich das die Amidseitenkette tragende Kohlenstoffatom in 3-Stellung des Ringgerüstes und das den Rest R¹ tragende Kohlenstoffatom der Amidseitenkette. Die Verbindungen können somit in mehreren optisch aktiven stereoisomeren Formen oder als Racemat vorliegen. Gemäß der vorliegenden Erfindung können sowohl die racemischen Gemische als auch die isomerenreinen Verbindungen der Formel I verwendet werden.

Die erfindungsgemäß zur Behandlung von Durchblutungsstörungen im Magendarmbereich eingesetzten Verbindungen fallen unter den Umfang von in der europäischen Patentanmeldung EP 733 642 A1 (vergleiche deutsche Patentanmeldung Nr. 195 10 566.4) beschriebenen Benzazepin-, Benzoxazepin- und Benzothiazepin-N-essigsäurederivaten, welche in α-Stellung zu dem Stickstoff eine Oxogruppe enthalten und in 3-Stellung durch einen 1-(Carboxyalkyl)-cyclopentylcarbonyl-amino-Rest substituiert sind, und NEP-inhibitorische Wirkungen am Herzen besitzen. Die EP 733 642 A1 stellt einen Stand der Technik gemäß Art. 54(3) EPÜ dar.

Es wurde nun überraschenderweise gefunden, daß die erfindungsgemäß verwendete Gruppe der Verbindungen der Formel I eine die Durchblutung des mesenterialen Gefäßsystems fördernde Wirkung am Menschen und größeren Säugetieren besitzt und zur Behandlung und/oder Prophylaxe von mit einer verminderten gastrointestinalen Durchblutung verschiedenster Genese einhergehenden gastrointestinalen Beschwerden, insbesondere Angina abdominales, geeignet ist. Die Ursachen für eine verminderte gastrointestinale Durchblutung können vielfältiger Genese sein, z. B. ein erhöhter Gefäßwiderstand, beispielsweise bei Gefäßveränderungen aufgrund von Artheriosclerose oder Entzündungen der den Magendarmbereich versorgenden Gefäße, pathologische Veränderungen der Gefäßfunktionen, welche im Zusammenhang mit Diabetis und/oder Herzkrankheiten wie hypertensive Kardiomyopathie stehen können.

Bei vermindertem Blutfluß in der mesenterialen Arterie kommt es zu einer Blutunterversorgung in der Magendarmwand, welche ungenügend ist, um die für eine gut funktionierende gastrointestinale Motilität benötigte Durchblutung zu gewährleisten, und insbesondere nicht mehr ausreicht, um die nach Nahrungseinnahme erhöhten Anforderungen an Motilität, Sekretion und Absorption zu erfüllen. Eine unzureichende Bluversorgung der Darmwände kann sich in gastrointestinalen Symptomen äußern wie akuten oder chronischen Schmerzen im Bauchbereich bis hin zu akuten Anfällen von Angina abdominales, welche besonders heftig nach Nahrungsaufnahme auftreten können, sowie auch Blähungen, Konstipation oder Diarrhoe.

Die die Durchblutung des mesenterialen Gefäßsystems fördernde Wirkung der erfindungsgemäß verwendeten Verbindungen der Formel I wurde in pharmakologischen Tests in vivo an gesunden Ratten und an diabetischen Ratten durch Messung der Substanzwirkung auf den Blutfluß in der Mesenterialaterie nachgewiesen.

### Beschreibung der Testmethode

Die Tests wurden an erwachsenen männlichen Wistar Ratten mit einem Anfangskörpergewicht von 250 - 270 g durchgeführt. Die Tiere wurden in 4 Gruppen von je 10 Tieren eingeteilt. In zwei der Gruppen wurde in den Tieren durch einmalige i.p.-Injektion von Streptozotocin (Dosis 65 mg/kg) Diabetis induziert.

Während der anschließenden 8-wöchigen Testperiode stand allen Tieren Trinkwasser unbegrenzt zur Verfügung und sie erhielten ein Standard Nagetierfutter.

Je eine Gruppe von gesunden Ratten und von diabetischen Ratten erhielten eine tägliche orale Dosis der Testsubstanz (30 mg/kg Körpergewicht) mit dem Futter.

Nach Ablauf der 8 Wochen wurden die Tiere durch i.p.-Injektion von Pentobarbital (45 mg/kg Körpergewicht) narkotisiert, tracheotomiert und nach Laparotomie in der Mittellinie wurde der Hauptstamm der Mesenterialaterie freigelegt um eine Blutflußmeßsonde an das Gefäß anzubringen. Der intestinale Blutfluß im Ruhezustand wurde mittels eines kalibrierten Sensors mit einer Ultraschall-Methode zur Messung von Durchflußzeitänderungen (ultrasonic transit time shift technique) bestimmt, welche das Blutflußvolumen in ml/Minute mißt.

In dieser Testmethode führte die Behandlung mit der Substanz des nachfolgenden Beispiels 6 sowohl in den gesunden Ratten als auch in den diabetischen Ratten zu einer signifikanten Erhöhung des mesenterialen Blutflusses im Vergleich zu den jeweiligen nicht mit Testsubstanz behandelten Kontrollgruppen, wie aus der nachfolgenden Tabelle A ersichtlich ist.

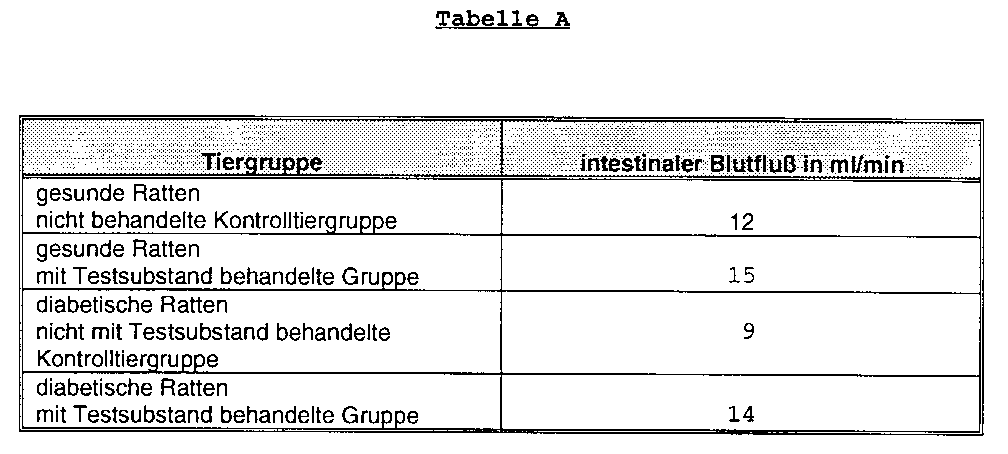

Aufgrund ihrer vorstehend beschriebenen Wirkung sind die Verbindungen der Formel I geeignet als Arzneimittel für größere Säugetiere, insbesondere Menschen, zur Behandlung und/oder Prophylaxe von Durchblutungsstörungen im Magendarmbereich und dadurch bedingten Krankheitszuständen, insbesondere Angina abdominales. Hierbei werden Dicarbonsäuren der Formel I und deren Salze zweckmäßig in parenteral, insbesondere i. v., applizierbaren Arzneiformen und Mono- oder Diester der Formel I zweckmäßig in oral applizierbaren Arzneiformen eingesetzt. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester oder flüssiger Trägerstoffe wie z. B. Milchzukker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die erfindungsgemäß verwendeten Verbindungen der Formel I können nach den in der vorgenannten europäischen Patentanmeldung EP 733 642 A1 (bzw. in der deutschen Patentanmeldung Nr. 195 10 566.4) beschriebenen Methoden hergestellt werden.

So können die erfindungsgemäß verwendeten Verbindungen der Formel I und deren Salze erhalten werden, indem man in an sich bekannter Weise
Säuren der allgemeinen Formel II 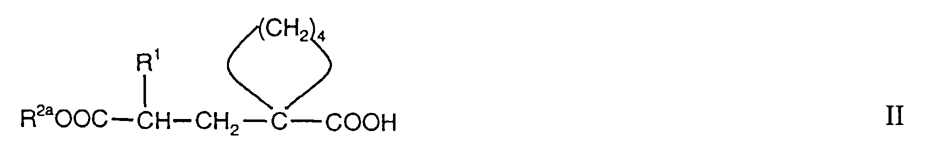 worin R¹ obige Bedeutung besitzt und R^{2a} eine Säureschutzgruppe bedeutet, oder deren reaktionsfähige Säurederivate mit Aminen der allgemeinen Formel III 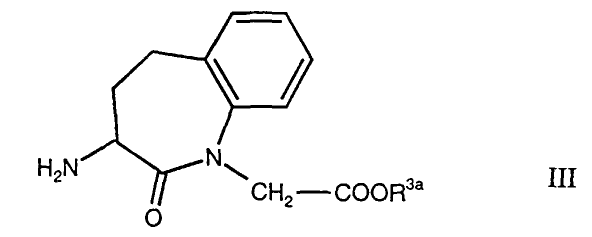 worin R^{3a} eine Säureschutzgruppe bedeutet, zu Amiden der allgemeinen Formel IV 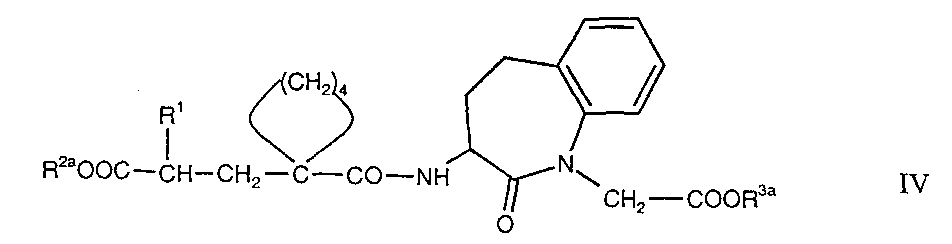 worin R¹, R^{2a} und R^{3a} obige Bedeutung besitzen, umsetzt und in den Verbindungen der Formel IV die Säureschutzgruppen R^{2a} und R^{3a}, sofern diese nicht eine gewünschte einen biolabilen Ester bildende Gruppe darstellen, gleichzeitig oder in beliebiger Reihenfolge nacheinander abspaltet und gewünschtenfalls jeweils die freiwerdende Säuregruppe mit einem Alkohol der allgemeinen Formel V oder einem entsprechenden reaktiven Derivat der allgemeinen Formel Va

R⁴-OH V

R⁴-X Va

worin R⁴ eine einen biolabilen Ester bildende Gruppe darstellt und X eine abspaltbare reaktive Gruppe bedeutet, verestert, und gewünschtenfalls erhaltene Säuren der Formel I in ihre physiologisch verträglichen Salze überführt oder Salze der Säuren der Formel I in die freien Säuren überführt.

Die Umsetzung der Säuren der Formel II mit den Aminen der Formel III zu den Amiden der Formel IV kann nach an sich zur Bildung von Amidgruppierungen durch Aminoacylierung üblichen Methoden ausgeführt werden. Als Acylierungsmittel können die Säuren der Formel II oder deren reaktionsfähige Derivate eingesetzt werden. Als reaktionsfähige Derivate kommen insbesondere gemischte Säureanhydride und Säurehalogenide in Frage. So können beispielsweise Säurechloride oder Säurebromide der Säuren der Formel II oder gemischte Ester der Säuren der Formel II mit organischen Sulfonsäuren, beispielsweise C1-C4-Alkansulfonsäuren wie z. B. Methansulfonsäure oder aromatischen Sulfonsäuren wie z. B. Benzolsulfonsäure oder durch C1-C4-Alkyl oder Halogen substituierten Benzolsulfonsäuren z. B. Toluolsulfonsäuren oder Brombenzolsulfonsäuren eingesetzt werden. Die Acylierung kann in einem unter den Reaktionsbedingungen inerten organischen Lösungsmittel, vorzugsweise bei Temperaturen zwischen -20 °C und Raumtemperatur erfolgen. Als Lösungsmittel eignen sich insbesondere halogenierte Kohlenwasserstoffe wie Dichlormethan oder aromatische Kohlenwasserstoffe wie Benzol oder Toluol oder cyclische Ether wie Tetrahydrofuran oder Dioxan oder Gemische dieser Lösungsmittel.

Die Acylierung kann zweckmäßig, insbesondere wenn als Acylierungsmittel ein gemischtes Anhydrid der Säuren der Formel II mit einer Sulfonsäure verwendet wird, in Gegenwart eines säurebindenden Reagenzes durchgeführt werden. Als säurebindende Mittel eignen sich in dem Reaktionsgemisch lösliche Basen, insbesondere organische Basen wie tert. Niederalkylamine und Pyridine wie z. B. Triethylamin, Tripropylamin, Pyridin, 4-Dimethylaminopyridin, 4-Diethylaminopyridin oder 4-Pyrrolidinopyridin. Im Überschuß eingesetzte organische Basen können gleichzeitig auch als Lösungsmittel dienen.

Vorteilhaft können gemischte Säureanhydride der Säuren der Formel II mit organischen Sulfonsäuren in situ durch Umsetzen der Säuren der Formel II mit einem Säurehalogenid, insbesondere dem Säurechlorid, der organischen Sulfonsäure erhalten werden und ohne Isolierung direkt weiter mit der Aminverbindung der Formel III umgesetzt werden.

Falls als Acylierungsmittel die Säuren der Formel II selbst eingesetzt werden, kann die Umsetzung der Aminoverbindungen der Formel III mit den Säuren der Formel II zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt Alkylcarbodiimide, z. B. Cycloalkylcarbodiimide wie Dicyclohexylcarbodiimid oder 1-Ethyl-3-[3-(dimethylamino)-propyl]-carbodiimid, Carbonyldiimidazol und N-Niederalkyl-2-halogenpyridiniumsalze, insbesondere Halogenide oder Tosylate, vorzugsweise N-Methyl-2-chlorpyridinium-jodid (siehe z. B. Mukaijama in Angewandte Chemie 91, Seiten 789 - 812). Die Umsetzung in Gegenwart eines Kopplungsreagenzes kann zweckmäßig bei Temperaturen von -30 bis +50 °C unter Benutzung von Lösungsmitteln wie halogenierten Kohlenwasserstoffen und/oder aromatischen Lösungsmitteln, gegebenenfalls in Gegenwart eines säurebindenden Amins durchgeführt werden.

Aus den durch Umsetzung der Verbindungen der Formel II mit den Verbindungen der Formel III erhaltenen Verbindungen der Formel IV können die Schutzgruppen R^{2a} und R^{3a}, sofern sie keine in den Verbindungen der Formel I gewünschten, einen biolabilen Ester bildende Gruppen darstellen, auf an sich bekannte Weise abgespalten werden.

Als Schutzgruppen R^{2a} und R^{3a} können an sich zum Schutz von Säurefunktionen übliche Schutzgruppen gewählt werden, die anschließend nach an sich bekannten Methoden wieder abgespalten werden. Geeignete Säureschutzgruppen sind beispielsweise bekannt aus McOmie, "Protective Groups in Organic Chemistry", Plenum Press und Greene, "Protective Groups in Organic Synthesis" Wiley Intersience Publication.

Sofern Verbindungen der Formel I hergestellt werden sollen, in denen R² und R³ identisch sind, werden zweckmäßigerweise identische Schutzgruppen R^{2a} und R^{3a} in den Ausgangsverbindungen II und III gewählt.

Sofern Verbindungen der Formel I hergestellt werden sollen, worin R² und R³ unterschiedliche Bedeutung besitzen, werden zweckmäßigerweise in den Ausgangsverbindungen II und III unterschiedliche Schutzgruppen gewählt, welche bei unterschiedlichen Bedingungen auf an sich bekannte Weise selektiv wieder abgespalten werden können. Als Beispiele dreier unter unterschiedlichen Bedingungen abspaltbarer Schutzgruppen seien genannt:
1. Methyl- oder Ethylester, welche unter basischen Bedingungen leicht gespalten werden, jedoch gegen saure Bedingungen oder Hydrogenolyse wesentlich stabiler sind,
2. tert.-Butylester, welche leicht durch Säuren gespalten werden können, jedoch gegen basische Bedingungen oder Hydrogenolyse wesentlich stabiler sind und
3. Benzylester, welche leicht hydrogenolytisch oder auch unter basischen Bedingungen gespalten werden können, jedoch gegenüber sauren Bedingungen wesentlich stabiler sind.

Falls beispielsweise Dicarbonsäure-Verbindungen der Formel I hergestellt werden sollen, worin R² und R³ beide Wasserstoff sind, werden als Schutzgruppen R^{2a} und R^{3a} bevorzugt sauer abspaltbare Schutzgruppen, beispielsweise die tert.-Butylgruppe, eingesetzt und die durch Umsetzung der Verbindungen der Formel II mit den Verbindungen der Formel III erhaltenen tert.-Butylester-Verbindungen der Formel IV anschließend durch Behandlung mit Säure gespalten. Die Spaltung kann z. B. durch Behandeln mit Trifluoressigsäure als solcher oder einer Trifluoressigsäurelösung in einem halogenierten Kohlenwasserstoff, beispielsweise Dichlormethan, oder durch Behandeln mit HCl-Gas in einem unter den Reaktionsbedingungen inerten organischen Lösungsmittel, beispielsweise Essigsäureethylester, erfolgen. Die Reaktion kann bei Temperaturen zwischen -25 °C und Raumtemperatur durchgeführt werden.

Falls beispielsweise Monocarbonsäure-Verbindungen der Formel I hergestellt werden sollen, worin R² eine einen biolabilen Ester bildende Gruppe bedeutet und R³ Wasserstoff ist, können als Ausgangsverbindungen der Formel II Verbindungen eingesetzt werden, in denen R^{2a} bereits die gewünschte einen biolabilen Ester bildende Gruppe, z. B. die Ethylgruppe, darstellt und als Schutzgruppe R^{3a} in den Verbindungen der Formel III Schutzgruppen, welche unter Bedingungen gespalten werden, unter denen die R²-OCO-Gruppe nicht gespalten wird. Falls die R²-OCO-Gruppe die relativ säurestabile Ethylestergruppe ist, eignen sich als Schutzgruppe R^{3a} beispielsweise die durch Säure abspaltbare tert.-Butylgruppe oder eine hydrogenolytisch abspaltbare Gruppe wie Benzyl.

Falls R^{2a} in den Verbindungen der Formel II eine säureempfindliche einen biolabilen Ester bildende Gruppe darstellt, wird als Schutzgruppe R^{3a} in den Verbindungen der Formel III zweckmäßig eine hydrogenolytisch abspaltbare Gruppe wie Benzyl gewählt und diese aus den durch Umsetzung der Verbindungen der Formel II mit den Verbindungen der Formel III hervorgegangen Verbindungen der Formel IV hydrogenolytisch abgespalten. Die Hydrogenolyse kann durch katalytische Hydrierung in Gegenwart eines Katalysators, vorzugsweise eines Pd/C-Katalysators in einem unter den Reaktionsbedingungen inerten organischen Lösungsmittel, beispielsweise einem C1-C4-Alkohol wie Ethanol oder einem C1-C4-Alkylester wie Essigsäureethylester erfolgen. Zweckmäßig wird die katalytische Hydrierung bei einem Wasserstoffdruck von 4 bis 5 bar bei Raumtemperatur durchgeführt.

Zur Herstellung von Verbindungen der Formel I, worin R² eine einen biolabilen Ester bildende Gruppe und R³ Wasserstoff bedeuten, können jedoch auch Ausgangsverbindungen der Formeln II und III mit unterschiedlichen Schutzgruppen R^{2a} und R^{3a} mit unterschiedlicher Reaktivität gewählt werden und aus den durch Umsetzung der Verbindungen der Formel II mit den Verbindungen der Formel III erhaltenen Verbindungen der Formel IV zunächst die Schutzgruppe R^{2a} unter Erhalt der Schutzgruppe R^{3a} abgespalten werden, dann in das Reaktionsprodukt der allgemeinen Formel IV' 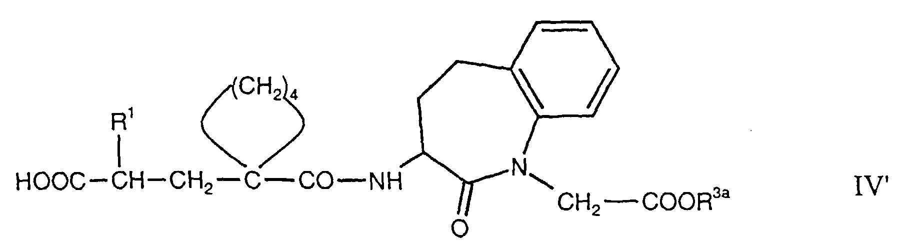 worin R¹ und R^{3a} obige Bedeutung besitzen, die gewünschte einen biolabilen Ester bildende Gruppe R² durch Umsetzung der freien Säuregruppe der Verbindung der Formel IV' mit einer Verbindung der Formel V oder Va eingefügt werden und anschließend aus den erhaltenen Verbindungen der Formel IV die Schutzgruppe R^{3a} abgespalten werden.

So kann z. B. aus Verbindungen der Formel IV, worin R^{2a} eine durch Säure abspaltbare Schutzgruppe, insbesondere die tert.-Butylgruppe, und R^{3a} eine säurestabile Schutzgruppe, z. B. Benzyl, bedeuten zunächst nur die Schutzgruppe R^{2a} sauer abgespalten werden. Die erhaltene Monocarbonsäure der Formel IV' kann dann nach an sich zur Esterbildung üblichen Methoden mit einem Alkohol der Formel V oder einer entsprechenden Verbindung der Formel Va verestert werden. Als abspaltbare reaktive Gruppen X in den Verbindungen der Formel Va eignen sich Halogene, insbesondere Chlor oder Brom, oder ein organischer Sulfonsäurerest, beispielsweise der Rest einer C1-C4-Alkansulfonsäure wie z. B. Methansulfonsäure oder von aromatischen Sulfonsäuren wie Benzolsulfonsäure oder durch C1-C4-Alkyl oder Halogen substituierte Benzolsulfonsäuren wie Toluolsulfonsäuren. Zur Veresterung können Alkohole der Formel V beispielsweise mit einer Säure der Formel IV' oder einem reaktiven Säurederivat dieser Säure auf an sich zur Acylierung von Alkoholen bekannte Weise umgesetzt werden. Die Umsetzung kann beispielsweise unter den für die Umsetzung von Verbindungen der Formel II mit Verbindungen der Formel III angegebenen Reaktionsbedingungen durchgeführt werden.

In analoger Weise können durch Auswahl entsprechender unterschiedlicher Schutzgruppen auch Verbindungen der Formel I hergestellt werden, worin R³ eine einen biolabilen Ester bildende Gruppe bedeutet und R² für Wasserstoff oder eine von R³ unterschiedliche einen biolabilen Ester bildende Gruppe bedeutet.

Bei den vorgehend beschriebenen Umsetzungen werden die chiralen Zentren in den Ausgangsverbindungen der Formeln II und III nicht verändert, so daß je nach Art der Ausgangsverbindungen isomerenreine Verbindungen der Formel I oder Isomerengemische erhalten werden können. Zur Herstellung von isomerenreinen und somit optisch einheitlichen Verbindungen der Formel I werden zweckmäßigerweise enantiomerenreine Verbindungen der Formel II mit enantiomerenreinen Verbindungen der Formel III umgesetzt. Falls eine enantiomerenreine Verbindung der Formel II mit einer racemischen Verbindung der Formel III oder eine racemische Verbindung der Formel II mit einer enantiomerenreinen Verbindung der Formel III umgesetzt werden, wird jeweils ein Gemisch aus zwei Diastereomeren erhalten, welches gewünschtenfalls auf an sich bekannte Weise aufgetrennt werden kann. Die Umsetzung von racemischen Verbindungen der Formel II mit racemischen Verbindungen der Formel III ergibt entsprechende Gemische aus 4 Isomeren, welche gewünschtenfalls auf an sich bekannte Weise aufgetrennt werden können.

Die Ausgangsverbindungen der Formel II können nach an sich bekannten Methoden erhalten werden, beispielsweise indem man Acrylsäurederivate der allgemeinen Formel VI 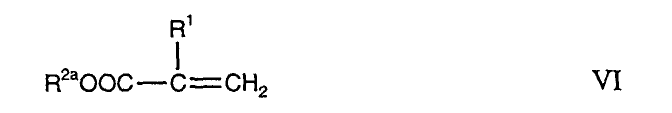 worin R^{2a} und R¹ obige Bedeutung besitzen, mit Cyclopentancarbonsäure der Formel VII 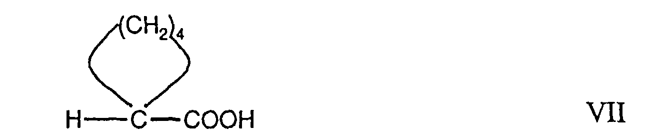 umsetzt. Die Reaktion kann auf an sich bekannte Weise unter den Bedingungen einer Michael-Addition in einem unter den Reaktionsbedingungen inerten organischen Lösungsmittel durch Reaktion der Cyclopentancarbonsäure mit einer starken, zur Bildung des Dianions der Cyclopentancarbonsäure befähigten, Base und anschließende Umsetzung mit dem Acrylesterderivat der Formel VI erfolgen. Als Lösungsmittel eignen sich Ether, insbesondere cyclische Ether wie beispielsweise Tetrahydrofuran. Als starke Basen eignen sich nicht-nucleophile, organische Alkalimetallamide wie z. B. Lithiumdiisopropylamid. Zweckmäßig wird die Cyclopentancarbonsäure in Tetrahydrofuran mit zwei Äquivalenten Lithiumdiisopropylamid umgesetzt und das Reaktionsgemisch anschließend mit der Verbindung der Formel VI weiter umgesetzt. Die Reaktionstemperatur kann zwischen -70 und 0 °C betragen.

Die Verbindungen der Formel II besitzen an dem den Rest R¹ tragenden Kohlenstoffatom ein Chiralitätszentrum und werden bei der Synthese in Form ihrer Racemate erhalten. Die optisch aktiven Verbindungen können aus den racemischen Gemischen in an sich bekannter Weise erhalten werden, z. B. durch chromatographische Trennung an chiralen Trennmaterialien oder durch Umsetzung mit geeigneten optisch aktiven Basen, z. B. α-Methylbenzylamin oder Pseudoephedrin, und anschließende Auftrennung in ihre optischen Antipoden durch fraktionierte Kristallisation der gewonnen Salze.

Acrylsäureesterderivate der Formel VI können auf an sich bekannte Weise erhalten werden, indem man (Di-C1-C4-alkylphosphono)-essigsäureester-Derivate der allgemeinen Formel VIII 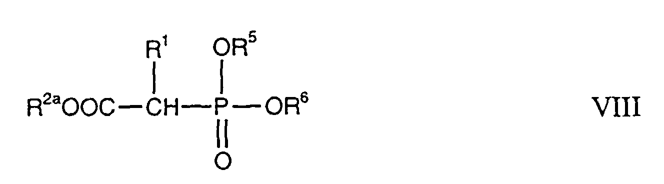 worin R^{2a} und R¹ obige Bedeutung besitzen und R⁵ und R⁶ je C1-C4-Alkyl, vorzugsweise Methyl oder Ethyl, bedeuten, mit Formaldehyd in einem unter den Reaktionsbedingungen inerten organischen Lösungsmittel unter basischen Bedingungen umsetzt. Beispielsweise können Verbindungen der Formel VIII mit Paraformaldehyd in einem Ether, vorzugsweise einem cyclischen Ether wie Tetrahydrofuran, in Gegenwart einer Base, vorzugsweise eines nicht-nukleophilen Alkalimetallalkoholates wie Kalium-tert.-butylat bei Temperaturen zwischen -20 und +30 °C umgesetzt werden.

Verbindungen der Formel VIII können auf an sich bekannte Weise erhalten werden, indem man Phosphonoessigsäure-Derivate der allgemeinen Formel IX 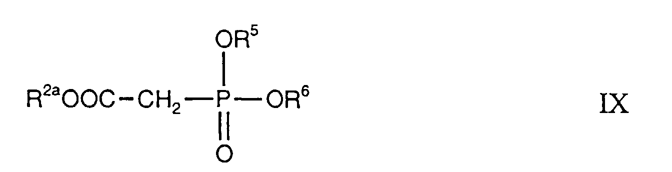 worin R^{2a}, R⁵ und R⁶ obige Bedeutung besitzen, mit Verbindungen der Formel X

R¹-X X

worin R¹ und X obige Bedeutung besitzen, umsetzt. Die Umsetzung kann unter zur Alkylierung üblichen Bedingungen in einem unter den Reaktionsbedingungen inerten polaren aprotischen organischen Lösungsmittel in Gegenwart einer Base bei Temperaturen zwischen 0 und 80 °C durchgeführt werden. Vorzugsweise werden Verbindungen der Formel X eingesetzt, worin X Halogen, insbesondere Brom oder Jod, oder Tosylat bedeutet. Als Lösungsmittel eignen sich beispielsweise Amide wie Dimethylformamid oder auch Ether. Als Basen eignen sich nicht-nukleophile Alkalimetallalkoholate wie beispielsweise Kalium-tert.-butylat.

Verbindungen der Formel VI können auch erhalten werden, indem man Malonsäurederivate der allgemeinen Formel XI 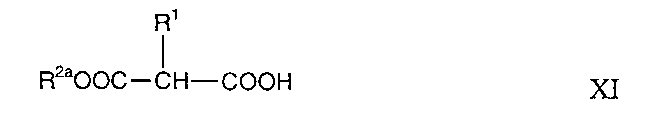 worin R^{2a} und R¹ obige Bedeutung besitzen, auf an sich bekannte Weise mit Formaldehyd unter basischen Bedingungen behandelt. So können Malonsäurederivate der Formel XI beispielsweise mit wäßriger Formaldehydlösung in Gegenwart eines sekundären organischen Amins, insbesondere Piperidin, bei Temperaturen zwischen 0 und 30 °C, vorzugsweise bei unterhalb Raumtemperatur liegenden Temperaturen, umgesetzt werden. Die Malonsäure-Derivate der Formel XI können auch mit Paraformaldehyd in Pyridin bei Temperaturen zwischen 40 bis 60 °C umgesetzt werden.

Die Malonsäuremonoester der Formel XI können erhalten werden, indem man Malonsäurediester der allgemeinen Formel XII

R^{2a}OOC-CH₂-COOR⁷ XII

worin R^{2a} obige Bedeutung besitzt und R⁷ C1-C4-Alkyl, insbesondere Methyl, oder Benzyl bedeutet, mit Verbindungen der Formel X umsetzt und die erhaltenen Malonsäurediester-Derivate der allgemeinen Formel XIII 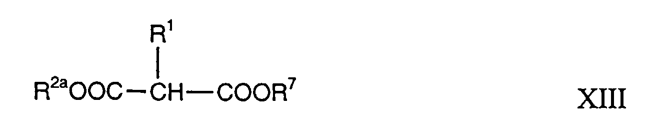 worin R¹, R^{2a} und R⁷ obige Bedeutung besitzen, durch partielle Hydrolyse in die entsprechenden Malonsäuremonoester-Derivate der Formel XI überführt.

Die Einführung des Restes R¹ in die Malonsäurediester der Formel XII kann auf an sich bekannte Weise durch Umsetzung der Ester der Formel XII mit einer Verbindung der Formel X in einem polaren aprotischen organischen Lösungsmittel, vorzugsweise Dimethylformamid, in Gegenwart einer Base, beispielsweise einem nicht-nukleophilen Alkalimetall-Alkoholat wie Kalium-tert.-butylat bei Temperaturen zwischen 0 °C und 80 °C erfolgen. Die Umsetzung kann beispielsweise unter den für die Umsetzung von Verbindungen der Formel VIII mit Verbindungen der Formel X angegebenen Bedingungen durchgeführt werden.

Die erhaltenen substituierten Malonsäurediester der Formel XIII können durch Abspaltung des Restes R⁷ auf an sich bekannte Weise in die entsprechenden Malonsäuremonoester der Formel XI überführt werden. Sofern die Schutzgruppe R^{2a} und der Rest R⁷ verschiedene Reste mit unterschiedlicher Reaktivität darstellen, werden für die Abspaltung des Restes R⁷ zweckmäßigerweise solche Bedingungen gewählt, unter denen der Rest R^{2a} nicht angegriffen wird. Sofern R⁷ Benzyl bedeutet, kann die Abspaltung auf an sich bekannte Weise hydrogenolytisch erfolgen. C1-C4-Alkylester R⁷ werden hydrolytisch, je nach Art des Alkylrestes unter sauren oder alkalischen Bedingungen auf an sich bekannte Weise abgespalten. Vorzugsweise stellt R⁷ Ethyl dar, welches durch alkalische Hydrolyse abgespalten werden kann. Hierzu können die Alkylester der Formel XIII in einem niederen Alkohol oder einem Gemisch aus einem niederen Alkohol mit Wasser mit einem Alkalimetallhydroxid, beispielsweise Kaliumhydroxid, behandelt werden. Sofern die Reste R^{2a} und R⁷ identisch sind, wird hierbei die Menge an Alkalimetallhydroxid so gering gehalten, daß nur eine partielle Hydrolyse eintritt.

Verbindungen der Formel III können auf an sich bekannte Weise erhalten werden, indem man Verbindungen der allgemeinen Formel XIV 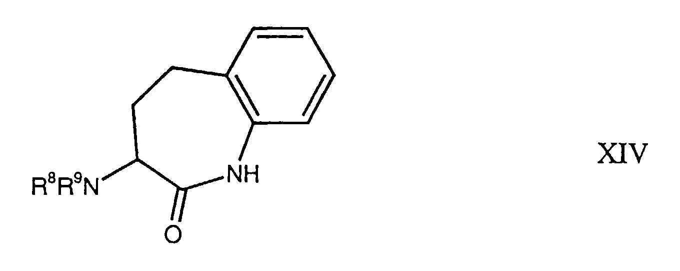 worin die R⁸R⁹N-Gruppe eine durch eine Aminoschutzgruppe geschützte Aminogruppe darstellt, mit Verbindungen der allgemeinen Formel XV

X-CH₂-COOR^{3a} XV

worin R^{3a} und X obige Bedeutung besitzen, umsetzt und in dem erhaltenen Reaktionsprodukt der allgemeinen Formel XVI 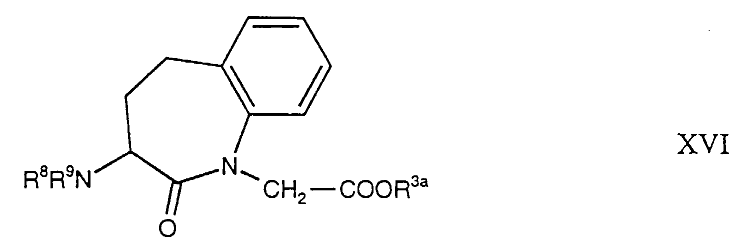 worin R^{3a} und die R⁸R⁹N-Gruppe obige Bedeutung besitzen, aus der R⁸R⁹N-Gruppe die freie Aminogruppe freisetzt. Die Umsetzung von Verbindungen der Formel XIV mit Verbindungen der Formel XV kann nach an sich zur Alkylierung von Amiden üblichen Methoden durchgeführt werden. Vorzugsweise werden Verbindungen der Formel XV eingesetzt, in welchen X für Halogen, vorzugsweise Brom oder Jod, steht. Die Umsetzung kann in einem polaren aprotischen organischen Lösungsmittel, beispielsweise Dimethylformamid oder einem cyclischen Ether wie Tetrahydrofuran in Gegenwart einer Base durchgeführt werden. Als Basen eignen sich nicht-nukleophile Basen wie beispielsweise Kalium-tert.-butylat. Gewünschtenfalls kann die Umsetzung auch in Gegenwart eines Alkalimetallhydroxides, z. B. Kaliumhydroxid, in einem Zweiphasensystem in Gegenwart eines Phasentransferkatalysators, beispielsweise eines Tetraniederalkylammoniumhalogenides wie Tetrabutylammoniumbromid, durchgeführt werden.

Anschließend kann in den erhaltenen Verbindungen der Formel XVI die Aminogruppe durch Abspaltung der Schutzgruppe in an sich bekannter Weise freigesetzt werden. Zum Schutz der Aminogruppe können die an sich zum Schutze von Aminogruppen bekannten leicht wieder abspaltbaren Schutzgruppen, beispielsweise die aus der Peptidchemie bekannten Schutzgruppen, eingesetzt werden. Geeignete Schutzgruppen sind z. B. bekannt aus E. McOmie "protective groups in organic chemistry" Plenum Press 1971. Beispielsweise eignen sich als Schutzgruppen die Phthalimidgruppe oder die tert.-Butoxycarbonylgruppe oder auch die Benzyloxycarbonylgruppe. Es müssen in Abhängigkeit der Bedeutung von R^{3a} jeweils Schutzgruppen gewählt werden, die anschließend unter Bedingungen abspaltbar sind, unter denen die Gruppe R^{3a} nicht angegriffen wird.

Die Verbindungen der Formel III enthalten an dem die Aminogruppe tragenden Kohlenstoffatom ein Chiralitätszentrum. Sofern von optisch reinen Ausgangsverbindungen der Formel XIV ausgegangen wird, werden optisch reine Verbindungen der Formel III erhalten. Sofern von racemischen Verbindungen der Formel XIV ausgegangen wird, werden auch racemische Verbindungen der Formel III erhalten. Racemische Gemische von Verbindungen der Formel III können auf an sich bekannte Weise in ihre optischen Isomeren aufgetrennt werden, beispielsweise durch chromatographische Trennung an chiralen Trennmaterialien oder durch Umsetzung mit geeigneten optisch aktiven Säuren, beispielsweise Weinsäure, und anschließende Auftrennung der optischen Antipoden durch fraktionierte Kristallisation der gewonnenen Salze. Zur Erhöhung der Ausbeute an dem gewünschten optischen Isomer kann bei der Umsetzung mit geeigneten optisch aktiven Säuren gleichzeitig mit oder nach der weitgehenden Fällung des Salzes des einen Isomeren mit der optisch aktiven Säure in dem Reaktionsgemisch eine Re-Racemisierung des in Lösung verbliebenen Isomeren durch Zusatz eines vorzugsweise aromatischen Aldehydes wie beispielsweise Benzaldehyd in Gang gesetzt werden. Dabei wird die Racemisierung an dem Chiralitätszentrum durch Iminbildung mit dem Aldehyd hervorgerufen.

Die Verbindungen der Formel XIV können auf an sich bekannte Weise erhalten werden, indem man in Verbindungen der allgemeinen Formel XVII 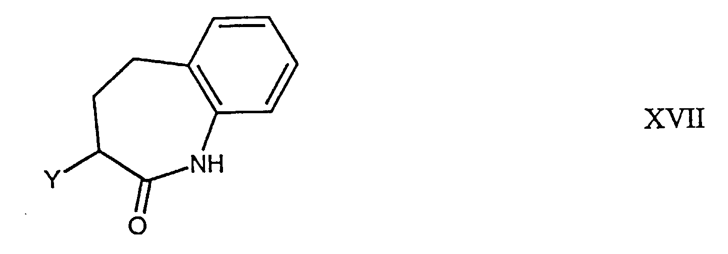 worin Y Halogen bedeutet, das Halogen Y durch die R⁸R⁹N-Gruppe auf an sich bekannte Weise ersetzt. Beispielsweise kann eine Verbindung der Formel XVII mit einem Alkalimetallsalz eines Amides R⁸R⁹NH, vorzugsweise Kalium-Phthalimid, umgesetzt werden. Die Umsetzung kann in einem unter den Reaktionsbedingungen inerten aprotischen organischen Lösungsmittel, vorzugsweise Dimethylformamid, bei Temperaturen zwischen 40 und 80 °C erfolgen.

Verbindungen der Formel XVII können auf an sich bekannte Weise durch Beckmann-Umlagerung von Oxim-Verbindungen der allgemeinen Formel XVIII 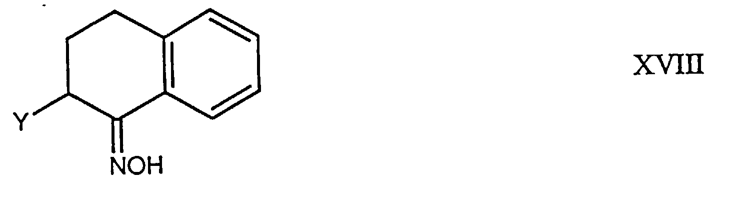 worin Y obige Bedeutung besitzt, erhalten werden, indem man Verbindungen der Formel XVIII unter Bedingungen einer Beckmann-Umlagerung mit einer Säure behandelt. Zweckmäßigerweise werden Verbindungen der Formel XVIII durch Behandeln mit Polyphosphorsäure bei Temperaturen zwischen 60 und 90 °C in die Verbindungen der Formel XVII umgelagert.

Oxime der Formel XVIII können ausgehend von dem cyclischen Keton der allgemeinen Formel XIX 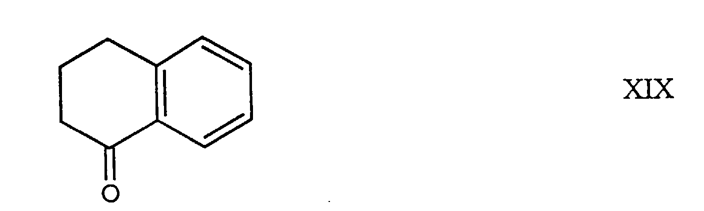 erhalten werden, indem man das Keton der Formel XIX zunächst zur Einführung des Restes Y mit Halogen behandelt und die erhaltenen halogenierten Ketone anschließend mit Hydroxylamin umsetzt. Zweckmäßigerweise können die α-Halogenierung des Ketones und die anschließende Oximbildung in einem Eintopfverfahren durchgeführt werden, wobei das Keton der Formel XIX zunächst in einem inerten organischen Lösungsmittel, beispielsweise einem niederen Alkohol wie Methanol, mit dem Halogen behandelt wird und anschließend dem Reaktionsgemisch Hydroxylamin zugeführt wird. Zweckmäßigerweise wird das Hydroxylamin in Form eines Hydroxylamin-Salzes, beispielsweise des Hydrochlorides, eingesetzt und dem Reaktionsgemisch etwas Wasser zugefügt. Das Verfahren kann bei Temperaturen zwischen 0 und 40 °C, vorzugsweise bei Raumtemperatur, durchgeführt werden.

Die nachfolgenden Beispiele sollen die Erfindung näher erläutern, jedoch deren Umfang in keiner Weise beschränken.

Die nachfolgenden Beispiele 1 und 2 beschreiben erfindungsgemäße pharmazeutische Zubereitungen enthaltend einen Wirkstoff der Formel I sowie die Herstellung solcher pharmazeutischer Zubereitungen.

### Beispiel 1:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure enthaltende Tabletten

Man stellte Tabletten in folgender Zusammensetzung pro Tablette her:

| | |
|---|---|
| (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure | 20 mg |
| Maisstärke | 60 mg |
| Milchzucker | 135 mg |
| Gelatine (als 10 %-ige Lösung) | 6 mg |

Der Wirkstoff, die Maisstärke und der Milchzucker wurden mit der 10 %-igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

### Beispiel 2:

### (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure enthaltende Injektionslösung.

Man stellte eine Injektionslösung mit folgender
Zusammensetzung pro 5 ml her:

| | |
|---|---|
| (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure | 10 mg |
| Na₂HPO₄·7H₂O | 43,24 mg |
| NaH₂PO₄·2H₂O | 7,72 mg |
| NaCl | 30,0 mg |
| gereinigtes Wasser | 4948,0 mg |

Die Feststoffe wurden in Wasser gelöst, die Lösung wurde sterilisiert und in Portionen von jeweils 5 ml in Ampullen abgefüllt.

Die nachfolgenden Beispiele sollen die Herstellung der Verbindungen der Formel I näher erläutern.

### Beispiel 3:

### 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.

A) Zu einer Lösung von 160,1 g Malonsäurediethylester in einem Liter Dimethylformamid wurden portionsweise 123,4 g Kalium-tert.-butylat bei einer Temperatur von 15 °C zugegeben. Das Reaktionsgemisch wurde 30 Minuten gerührt, dann wurde bei Raumtemperatur eine Lösung von 207,7 g Phenethylbromid in 200 ml Dimethylformamid zugetropft. Anschließend wurde das Reaktionsgemisch eine Stunde auf 60 °C gewärmt und wieder abkühlen gelassen. Das Dimethylformamid wurde unter vermindertem Druck abgedampft, der verbleibende Rückstand wurde in einem Gemisch aus Methyl-tert.butylether und Wasser aufgenommen. Die organische Phase wurde abgetrennt, mit Wasser gewaschen über Natriumsulfat getrocknet und eingedampft. Das als öliger Rückstand verbleibende Rohprodukt wurde durch Destillation unter vermindertem Druck gereinigt. Es wurden 202,5 g 2-Ethoxycarbonyl-4-phenyl-butansäure-ethylester erhalten, KP_{1,5} = 148 - 153 °C.
B) Zu einer Lösung von 23,6 g des vorstehend erhaltenen Diesterproduktes in 285 ml Ethanol wurde eine Lösung von 6,17 g Kaliumhydroxid in 76 ml Wasser unter Eiskühlung zugegeben. Das Reaktionsgemisch wurde mehrere Stunden bei Raumtemperatur gerührt. Anschließend wurde das Ethanol unter vermindertem Druck abgedampft und der Rückstand in einem Gemisch aus Methyl-tert.-butylether und Wasser aufgenommen. Die organische Phase wurde abgetrennt und verworfen und die wäßrige Phase wurde unter Eiskühlung mit verdünnter wäßriger Salzsäure angesäuert und anschließend mehrmals mit Methyl-tert-butylether extrahiert. Die vereinigten Methyl-tert.-butylether-Phasen wurden mit Wasser gewaschen, über Natriumsulfat getrocknet und unter vermindertem Druck eingedampft. Es wurden 20,2 g rohes öliges 2-Carboxy-4-phenylbutansäure-ethylester erhalten, welches ohne weitere Reinigung weiterverarbeitet wurde.
C) Zu 20,2 g des vorstehend erhaltenen Produktes wurden unter Eiskühlung nacheinander 11 ml 35 %-ige wäßrige Formaldehyd-Lösung und 9,23 ml Piperidin zugegeben. Das Reaktionsgemisch wurde mehrere Stunden bei Raumtemperatur gerührt, dann mit Methyl-tert.-butylether verdünnt, mit wäßriger Kaliumhydrogensulfat-Lösung und mit Wasser gewaschen, über Natriumsulfat getrocknet und eingedampft. Der Rückstand wurde unter vermindertem Druck getrocknet. Es wurden 14,8 g α-(2-Phenylethyl)-acrylsäureethylester erhalten.
D) Unter Stickstoffatmosphäre wurden 25,2 ml Diisopropylamin in 150 ml absolutem Tetrahydrofuran gelöst und auf -35 °C abgekühlt. Zu der Lösung wurden 100 ml einer 1,6-normalen Butyllithium-Lösung in n-Hexan zugetropft. Dann wurde das Reaktionsgemisch 30 Minuten bei 0 °C gerührt und anschließend wurde eine Lösung von 8,1 ml Cyclopentancarbonsäure in 20 ml absolutem Tetrahydrofuran zugetropft. Das Reaktionsgemisch wurde 2 Stunden bei 0 °C gerührt. Dann wurde eine Lösung von 16,8 g des unter C) erhaltenen Acrylesters in 20 ml absolutem Tetrahydrofuran zugetropft, das Reaktionsgemisch 2 Stunden bei 0 °C und anschließend mehrere Stunden bei -15 °C stehengelassen. Zur Aufarbeitung wurde das Reaktionsgemisch mit 10 %-iger wäßriger Salzsäure-Lösung angesäuert und mit n-Hexan extrahiert. Die organische Phase wurde siebenmal mit halbgesättigter wäßriger Natriumbicarbonat-Lösung und einmal mit Wasser gewaschen, über Natriumsulfat getrocknet und unter vermindertem Druck eingedampft. Das als Rückstand erhaltene Rohprodukt wurde durch Flash-Chromatographie an Kieselgel unter Verwendung von n-Hexan/ Essigsäureethylester (8:2) gereinigt. Es wurden 19,6 g reines 1-[2'-(Ethoxycarbonyl)-4'-phenyl-butyl]-cyclopentan-1-carbonsäure mit einem Schmelzpunkt von 68 bis 69 °C erhalten.
E) Zu einer Lösung von 100 g α-Tetralon in 820 ml Methanol wurden unter Eiskühlung 108,3 g Brom langsam zugetropft. Danach wurde das Reaktionsgemisch 30 Minuten bei Raumtemperatur gerührt und dann wurden zunächst 122,4 g Hydroxylamin-hydrochlorid und anschließend 110 ml Wasser bei Raumtemperatur zugesetzt. Das Gemisch wurde 3 Tage bei Raumtemperatur gerührt. Dann wurden weitere 493 ml Wasser zugefügt, wobei nach einer Stunde ein weißer Niederschlag ausfiel. Das Reaktionsgemisch wurde weitere 3 Tage gerührt und dann auf 5 °C abgekühlt. Der Niederschlag wurde abgesaugt, mit Wasser gewaschen und bei 40 °C unter vermindertem Druck getrocknet. Es wurden 136,7 g 2-Brom-3,4-Dihydronaphthalin-1(2H)-on-oxim mit einem Schmelzpunkt von 130 bis 132 °C erhalten.
F) 79,5 g des vorstehend erhaltenen Oxims wurden portionsweise zu 452 g auf 80 °C erwärmter Polyphosphorsäure gegeben und das Reaktionsgemisch wurde 18 Stunden bei 80 °C gerührt. Anschließend wurde vorsichtig mit 710 ml Wasser verdünnt und das Gemisch 2 Stunden bei Raumtemperatur gerührt. Der entstandene Niederschlag wurde abgesaugt, mit Wasser, wäßriger Natriumbicarbonat-Lösung, nochmals mit Wasser und abschließend mit Methyl-tert.-butylether gewaschen und über Kaliumhydroxid bei einer Temperatur von 60 °C getrocknet. Es wurden 66,6 g 3-Brom-4,5-dihydro-1H-1-benzazepin-2(3H)-on mit einem Schmelzpunkt von 168 bis 170 °C erhalten.
G) 80 g des vorstehend erhaltenen Produktes wurden in 140 ml Dimethylformamid suspendiert. Die Suspension wurde mit einer Lösung von 72,6 g Kaliumphthalimid in 205 ml Dimethylformamid versetzt und anschließend 16 Stunden bei 60 °C gerührt. Zur Aufarbeitung wurde auf Raumtemperatur abgekühlt, und es wurden langsam 800 ml Wasser zugetropft und das Gemisch 2 Stunden unter Eiskühlung gerührt. Der entstandene Kristallbrei wurde abgesaugt, und erst mit einem Wasser/Dimethylformamid-Gemisch und dann mit Methyl-tert.-butyl-ether gewaschen und anschließend 2 Tage bei 60 °C unter vermindertem Druck getrocknet. Es wurden 73,3 g 4,5-Dihydro-3-phthalimido-1H-1-benzazepin-2(3H)-on mit einem Schmelzbereich von 185 bis 195 °C erhalten.
H) Zu einer Suspension von 27 g des vorstehend erhaltenen Produktes in 90 ml Dimethylformamid wurde unter Eiskühlung eine Lösung von 12,3 g Kalium-tert.-butylat in 40 ml Dimethylformamid gegeben. Nach 30-minütigem Rühren unter Eiskühlung wurden 20,7 g Bromessigsäure-tert.-butylester über eine Stunde verteilt bei 0 bis 5 °C zugetropft. Es wurde eine Stunde bei 0 °C gerührt. Dann wurde das Reaktionsgemisch auf 40 °C erwärmt, und es wurden 164 ml Wasser innerhalb von 3 Stunden zugetropft und noch eine Stunde bei 30 °C gerührt. Dann wurde die wäßrige Lösung von dem gebildeten Niederschlag abdekantiert und der verbleibende feste Rückstand wurde aus Methyl-tert.-butylether kristallisiert. Die gebildeten Kristalle wurden abgesaugt, mit Wasser und mit Methyl-tert.-butylether gewaschen und unter vermindertem Druck bei 60 °C getrocknet. Es wurden 26,3 g 2,3,4,5-Tetrahydro-2-oxo-3-phthalimido-1H-1-benzazepin-1-essigsäure-tert.-butylester mit einem Schmelzpunkt von 194 - 197 °C erhalten.
I) 7 g des vorstehend erhaltenen Esters wurden innerhalb von 5 Minuten zu 13,8 ml auf 80 °C erwärmtem Ethanolamin gegeben. Nach 5 Minuten hatte sich eine klare Lösung gebildet, welche auf Raumtemperatur abgekühlt und mit 105 ml Toluol verdünnt wurde. Die Lösung wurde mit 140 ml 5 %-iger wäßriger Natriumchlorid-Lösung ausgeschüttelt, die organische Phase wurde abgetrennt, über Natriumsulfat getrocknet und eingedampft. Der verbleibende Rückstand wurde aus Methyl-tert.-butylether kristallisiert. Es wurden 4,0 g 3-Amino-2,3,4,5-tetrahydro-2-oxo-1H-1-benzazepin-1-essigsäure-tert.-butylester mit einem Schmelzpunkt von 117 bis 118 °C erhalten.
J) 2,9 g des vorstehend erhaltenen Amins und 3,2 g der vorstehend unter D) erhaltenen Säure wurden in 100 ml Dichlormethan gelöst. Zu dem Reaktionsgemisch wurden 2,2 ml N-Methylmorpholin, 1,27 g Hydroxybenzotriazol und 3,81 g N-Ethyl-N-(3-dimethylaminopropyl)-carbodiimid-hydrochlorid unter Eiskühlung zugefügt. Dann wurde das Reaktionsgemisch 1 Stunde bei Raumtemperatur gerührt. Zur Aufarbeitung wurde das Reaktionsgemisch mit Dichlormethan verdünnt und nacheinander mit Wasser, wäßriger Kaliumhydrogensulfat-Lösung, Wasser, wäßriger Natriumbicarbonatlösung und erneut mit Wasser gewaschen. Die organische Phase wurde sodann über Natriumsulfat getrocknet und das Lösungsmittel wurde unter vermindertem Druck abgedampft. Das so erhaltene Rohprodukt wurde mittels Säulenchromatographie an Kieselgel unter leicht erhöhtem Druck (= Flash-Chromatographie) unter Verwendung von n-Hexan/Essigsäureethylester gereinigt, wobei der Essigsäureester-Anteil des Elutionsmittels während der Elution von anfangs 1:9 auf 3:7 erhöht wurde. Es wurden 5,4 g der reinen Titelverbindung als öliges Produkt erhalten.
   IR-Spektrum (als Film): 3400 cm⁻¹, 1725 cm⁻¹, 1660 cm⁻¹

### Beispiel 4:

### 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.

5 g 3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 3) wurden in 16 ml Trifluoressigsäure gelöst. Die Lösung wurde 3 Stunden bei Raumtemperatur gerührt. Zur Aufarbeitung wurde die Trifluoressigsäure unter vermindertem Druck abgedampft. Der verbleibende Rückstand wurde in Dichlormethan gelöst und die Lösung wurde mit Wasser neutral gewaschen. Anschließend wurde die organische Phase über Natriumsulfat getrocknet und unter vermindertem Druck eingedampft. Der verbleibende Rückstand wurde mehrfach mit n-Hexan verrührt und jeweils wieder zur Trockene eingedampft. Es wurden 3,4 g der Titelverbindung als fester Schaum mit einem Schmelzbereich von 81 bis 104 °C erhalten.

### Beispiel 5:

### (3S,2'R)-3-{1-[2'-Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.

A) 30,5 g 1-[2'-(Ethoxycarbonyl)-4'-phenyl-butyl]-cyclopentan-1-carbonsäure (Herstellung siehe Beispiel 3D)) und 11,6 g L-(-)-a-Methylbenzylamin wurden in Ethanol unter Erwärmung gelöst. Das Reaktionsgemisch wurde 12 Stunden im Kühlschrank gekühlt, dann wurde der ausgefallene Kristallbrei abgesaugt, getrocknet und mehrmals (bis zur Drehwertkonstanz) aus Ethanol umkristallisiert und anschließend unter vermindertem Druck getrocknet. Es wurden 17,7 g eines α-Methylbenzylammoniumsalzes der vorstehenden Säure mit einem Schmelzpunkt von 118 bis 121 °C erhalten, optischer Drehwert [α]²⁰_{D} = +5,6° (c = 0,5 in Methanol).
   Zur Freisetzung der Säure wurde dieses Salz in einem Wasser/Dichlormethan-Gemisch aufgenommen und das Gemisch wurde mit wäßriger Kaliumhydrogensulfat-Lösung sauergestellt. Die organische Phase wurde abgetrennt und die wäßrige Phase noch dreimal mit Dichlormethan extrahiert. Die vereinigten organischen Extrakte wurden mit Wasser gewaschen, über Natriumsulfat getrocknet und unter vermindertem Druck eingedampft. Der verbleibende Rückstand wurde getrocknet. Es wurden 11,2 g reine (2'R)-1-[2'-(Ethoxycarbonyl-4'-phenylbutyl]-cyclopentan-1-carbonsäure erhalten, optischer Drehwert [α]²⁰_{D} = +7,4° (c = 0,651 in Methanol).
B) Zu einer auf 65 °C erhitzten Lösung von 24,5 g des racemischen 3-Amino-2,3,4,5-tetrahydro-2-oxo-1H-1-benzazepin-1-essigsäure-tert.-butylester (Herstellung siehe Beispiel 1I) wurde eine Lösung von 12,65 g L-(+)-Weinsäure in 54 ml auf 65 °C erhitztem Ethanol gegeben. Das Reaktionsgemisch wurde eine Stunde bei Raumtemperatur gerührt. Dann wurde eine Lösung von 1,72 ml Benzaldehyd in 1,3 ml Ethanol hinzugetropft. Die erhaltene Suspension wurde 14 Stunden bei 80 °C am Rückfluß gekocht und dann auf Raumtemperatur abgekühlt. Der entstandene kristalline Niederschlag wurde abgesaugt, in 80 ml Ethanol aufgenommen und erneut 8 Stunden am Rückfluß gekocht. Dann wurde auf Raumtemperatur abgekühlt und die Kristalle wurden abgesaugt und unter vermindertem Druck bei 50 °C getrocknet. Es wurden 23,6 g weinsaures Salz mit einem Schmelzpunkt von 195 bis 196 °C und einem optischen Drehwert [α]²⁰_{D} von -152° (c=0,5 in Methanol) erhalten.
   Zur Freisetzung der Base wurden 23,6 g des weinsauren Salzes in einem Gemisch aus 250 ml Wasser und 108 ml Dichlormethan unter Rühren auf 0 °C abgekühlt und durch Zusatz von wäßriger Ammoniak-Lösung auf pH 9,6 eingestellt. Die organische Phase wurde abgetrennt, die wäßrige Phase nochmals mit 30 ml Dichlormethan extrahiert und die organischen Phasen vereinigt, über Natriumsulfat getrocknet und unter vermindertem Druck eingeengt. Der verbleibende Rückstand wurde aus Methyl-tert.-butylether kristallisiert und unter vermindertem Druck getrocknet. Es wurden 12,2 g (3S)-3-Amino-2,3,4,5-tetrahydro-2-oxo-1H-1-benzazepin-1-essigsäure-tert.-butylester mit einem Schmelzpunkt von 113 bis 115 °C und einem optischen Drehwert [α]²⁰_{D} von -276,2° (c=0,5 in Methanol) erhalten.
C) 5,4 g der vorstehend unter A) hergestellten Säure wurden in 60 ml trockenem Dichlormethan gelöst. Die Lösung wurde mit 2,33 ml Triethylamin versetzt und auf -20 °C abgekühlt. Dann wurde langsam eine Lösung von 1,31 ml Methansulfonsäurechlorid in 5 ml trockenem Dichlormethan zugetropft. Nach 15-minütigem Rühren wurde eine Lösung von 4,8 g des vorstehend unter B) erhaltenen Amins und 2,33 ml Triethylamin in 60 ml Dichlormethan zugetropft. Anschließend wurde das Reaktionsgemisch 1 Stunde bei Raumtemperatur gerührt. Zur Aufarbeitung wurde das Reaktionsgemisch in Wasser gegeben, die organische Phase abgetrennt und mit wäßriger Kaliumhydrogensulfat-Lösung und anschließend mit Wasser gewaschen, über Natriumsulfat getrocknet, filtriert und unter vermindertem Druck eingeengt. Das verbleibende Rohprodukt wurde durch Flash-Chromatographie an 500 g Kieselgel unter Verwendung von n-Hexan/Essigsäureethylester (7:3) gereinigt. Nach Trocknen unter vermindertem Druck wurden 9,5 g reine Titelverbindung als Öl erhalten, optischer Drehwert [α]²⁰_{D} = -115,2° (c = 0,463 in Methanol).

### Beispiel 6:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.

9,4 g (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 5) wurden unter Eiskühlung in 15 ml Dichlormethan gelöst. Die Lösung wurde mit 31 ml Trifluoressigsäure versetzt und das Reaktionsgemisch wurde ca. 12 Stunden bei 4 °C im Kühlschrank gehalten. Zur Aufarbeitung wurden das Dichlormethan und die Trifluoressigsäure unter vermindertem Druck abgedampft. Das erhaltene Rohprodukt wurde in Essigsäureethylester aufgenommen, mit Wasser, verdünnter wäßriger Natriumbicarbonatlösung und erneut mit Wasser gewaschen. Die organische Phase wurde abgetrennt, über Natriumsulfat getrocknet und unter vermindertem Druck eingedampft. Der verbleibende Rückstand wurde durch Flash-Chromatographie an Kieselgel gereinigt, wobei als Elutionsmittel zunächst Dichlormethan und dann Dichlormethan/Methanol (95:5) verwendet wurde. Das erhaltene Produkt wurde 2 Tage bei 80 °C unter vermindertem Druck getrocknet. Es wurden 7,3 g der reinen Titelverbindung als fester Schaum mit einem Schmelzpunkt von 71 bis 74 °C erhalten, optischer Drehwert [α]²⁰_{D} = -131,0° (c = 0,5 in Methanol).

### Beispiel 7:

### 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.butylester.

A) 118 g Dimethylphosphonoessigsäure-tert.-butylester wurden unter Stickstoffatmosphäre in 875 ml trockenem Dimethylformamid gelöst. Zu der Lösung wurden 58,9 g Kalium-tert.-butylat unter Eiskühlung gegeben. Anschließend wurde das Reaktionsgemisch für kurze Zeit auf 60 °C erhitzt und dann auf Raumtemperatur abkühlen gelassen. Zu dem Reaktionsgemisch wurde eine Lösung von 104,9 g Phenethylbromid in 110 ml Dimethylformamid zugetropft. Dann wurde das Reaktionsgemisch für 2 Stunden auf 60 °C erhitzt. Zur Aufarbeitung wurde das Dimethylformamid unter vermindertem Druck weitgehend abgedampft und der verbliebene Rückstand wurde in Methyl-tert.-butylether gelöst. Die Lösung wurde mit wäßriger Kaliumhydrogensulfat-Lösung angesäuert. Dann wurde die organische Phase abgetrennt, mit Wasser gewaschen, über Natriumsulfat getrocknet und unter vermindertem Druck eingedampft. Das erhaltene Rohprodukt wurde durch Flash-Chromatographie an 3 kg Kieselgel unter Verwendung von Dichlormethan/Methyl-tert.-butylether (4:1) als Elutionsmittel gereinigt. Es wurden 105,1 g reines 2-(Dimethylphosphono)-4-phenyl-n-buttersäure-tert.-butylester als öliges Produkt erhalten.
B) 105,1 g des vorstehend erhaltenen Produktes wurden unter Stickstoffatmosphäre in 705 ml trockenem Tetrahydrofuran gelöst. Zu der Lösung wurden 28,4 g Paraformaldehyd gegeben. Dann wurde langsam eine Lösung von 32,5 g Kalium-tert.-butylat in 100 ml Tetrahydrofuran zugetropft. Anschließend wurde das Reaktionsgemisch 1 Stunde gerührt. Zur Aufarbeitung wurde das Reaktionsgemisch mit kalter wäßriger Kaliumhydrogensulfat-Lösung angesäuert und mit Methyl-tert.-butylether verdünnt. Dann wurde die organische Phase abgetrennt, mit Wasser gewaschen, über Natriumsulfat getrocknet und unter vermindertem Druck eingeengt. Das erhaltene Rohprodukt wurde durch Flash-Chromatographie an 700 g Kieselgel unter Verwendung von n-Hexan/Essigsäureethylester (9:1) gereinigt. Es wurden 47,0 g α-(Phenethyl)-acrylsäure-tert.-butylester als farbloses Öl erhalten.
C) Zu einer auf -50 °C abgekühlten Lösung von 50,2 ml Diisopropylamin in 450 ml absolutem Tetrahydrofuran wurden 200 ml einer 1,6-molaren Lösung von Butyllithium in n-Hexan getropft und das Reaktionsgemisch wurde weitere 30 Minuten bei 0 °C gehalten. Anschließend wurde bei dieser Temperatur eine Lösung von 16,2 ml Cyclopentancarbonsäure in 40 ml absolutem Tetrahydrofuran zugetropft. Das Reaktionsgemisch wurde weitere 2 Stunden bei 0 °C gerührt. Dann wurde zu dem Gemisch langsam eine Lösung von 38 g des vorstehend unter B) erhaltenen Produktes in 50 ml absolutem Tetrahydrofuran zugegeben. Das Reaktionsgemisch wurde weitere 2 Stunden bei 0 °C gerührt und dann noch mehrere Stunden bei -15 °C stehengelassen. Zur Aufarbeitung wurde das Reaktionsgemisch unter Eiskühlung mit gesättigter wäßriger Kaliumhydrogensulfat-Lösung angesäuert und dreimal mit n-Hexan extrahiert. Die vereinigten organischen Phasen wurden siebenmal mit halbgesättigter wäßriger Natriumbicarbonat-Lösung und anschließend mit Wasser gewaschen, dann über Natriumsulfat getrocknet und unter vermindertem Druck eingedampft. Das erhaltene ölige Rohprodukt wurde aus eiskaltem n-Hexan kristallisiert. Es wurden 41,9 g reine kristalline 1-[2'-(tert.-Butoxycarbonyl)-4'-phenyl-butyl]-cyclopentan-1-carbonsäure mit einem Schmelzpunkt von 75 bis 77 °C erhalten.
D) 3,3 g des vorstehend erhaltenen Produktes, 2,7 g 3-Amino-2,3,4,5-tetrahydro-2-oxo-1H-1-benzazepin-1-essigsäure-tert.-butylester (Herstellung siehe Beispiel 3I)), 1,53 ml N-Methylmorpholin und 1,18 g Hydroxybenzotriazol wurden unter Stickstoffatmosphäre in 93 ml absolutem Dichlormethan gelöst. Zu dieser Lösung wurden unter Eiskühlung 3,52 g N-Ethyl-N'-(3-dimethylaminopropyl)-carbodiimid-hydrochlorid zugefügt. Dann wurde das Reaktionsgemisch 2 Stunden unter Eiskühlung gerührt. Zur Aufarbeitung wurde das Reaktionsgemisch nacheinander mit Wasser, wäßriger Kaliumhydrogensulfat-Lösung, Wasser, wäßriger Natriumbicarbonat-Lösung und wiederum Wasser gewaschen. Die organische Phase wurde über Natriumsulfat getrocknet und unter vermindertem Druck eingeengt. Das verbleibende Rohprodukt wurde durch Flash-Chromatographie an 200 g Kieselgel unter Verwendung von n-Hexan/Essigsäureethylester (7:3) als Elutionsmittel gereinigt und aus Methyl-tert.-butylether kristallisiert. Es wurden 4,2 g der reinen Titelverbindung mit einem Schmelzpunkt von 110 bis 114 °C erhalten.

### Beispiel 8:

### 3-[1-(2'-Carboxy-4'-phenyl-butyl)-cyclopentan-1-carbonylamino]-2,3,4,5-tetrahydro-2-oxo-1H-1-benzazepin-1-essigsäure.

4,1 g 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.-butylester (Herstellung siehe Beispiel 7) wurden in 13 ml Trifluoressigsäure bei einer Temperatur von 4 °C unter Feuchtigkeitsausschluß gelöst. Die erhaltene Lösung wurde weitere 3 Stunden bei dieser Temperatur gerührt. Zur Aufarbeitung wurde das Reaktionsgemisch unter vermindertem Druck eingeengt. Zur völligen Entfernung von Trifluoressigsäure wurde der Rückstand mehrfach mit Dichlormethan versetzt und wieder eingedampft. Der erhaltene Rückstand wurde dann in Dichlormethan gelöst, die Lösung mit Wasser gewaschen, über Natriumsulfat getrocknet und unter vermindertem Druck eingeengt. Das als Rückstand verbleibende Rohprodukt wurde aus Dichlormethan kristallisiert. Es wurden 2,7 g der reinen Titelverbindung mit einem Schmelzpunkt von 178 bis 183 °C erhalten.

### Beispiel 9:

### 3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-benzylester.

A) 10,5 g 3-Amino-2,3,4,5-tetrahydro-2-oxo-1H-1-benzazepin-1-essigsäure-tert.-butylester (Herstellung siehe Beispiel 3 I), 8,25 g p-Toluolsulfonsäure-Hydrat und 20,1 ml Benzylalkohol wurden in 174 ml Toluol gegeben. Das Reaktionsgemisch wurde 4 Stunden am Wasserabscheider gekocht, wobei ein ursprünglich ausgefallener Niederschlag langsam in Lösung ging. Danach wurde das Toluol unter vermindertem Druck abgezogen und der verbleibende Rückstand wurde mit Methyl-tert.-butylether gerührt und dann abfiltriert. Der so erhaltene feste Rückstand wurde in Dichlormethan gelöst und die Lösung wurde durch Zugabe von wäßriger NatriumcarbonatLösung unter Eiskühlung alkalisch gestellt. Danach wurde die Dichlormethanphase abgetrennt, mit Wasser gewaschen, über Natriumsulfat getrocknet und eingedampft. Das erhaltene Rohprodukt wurde zur Reinigung aus Methyl-tert.-butyl-ether umkristallisiert. Es wurden 8,2 g 3-Amino-2,3,4,5-tetrahydro-2-oxo-1H-1-benzazepin-1-essigsäurebenzylester mit einem Schmelzpunkt von 105 bis 107 °C erhalten.
B) 12,8 g des vorstehend erhaltenen Produktes wurden mit 13,7 g 1-[2'-(tert.-Butoxycarbonyl)-4'-phenyl-butyl]-cyclopentan-1-carbonsäure (Herstellung siehe Beispiel 5C)) nach der in Beispiel 5C) beschriebenen Methode umgesetzt. Das Reaktionsgemisch wurde wie in Beispiel 5C) beschrieben, aufgearbeitet. Es wurden 19,3 g der Titelverbindung mit einem Schmelzpunkt von 118 bis 123 °C erhalten.

### Beispiel 10:

### 3-[1-(2'-Carboxy-4'-phenyl-butyl)-cyclopentan-1-carbonylamino]-2,3,4,5-tetrahydro-2-oxo-1H-1-benzazepin-1-essigsäure-benzylester.

15 g 3-{1-[2'-tert.-Butoxycarbonyl)-4'-phenyl-butyl]-cyclopentan-1-carbonylamino}-2,3,4,5-tetrahydro-2-oxo-1H-1-benzazepin-1-essigsäurebenzylester (Herstellung siehe Beispiel 9) wurden nach der in Beispiel 8 beschriebenen Methode mit 56 ml Trifluoressigsäure umgesetzt. Das Reaktionsgemisch wurde wie in Beispiel 8 beschrieben aufgearbeitet und das erhaltene Rohprodukt wurde aus Methyl-tert.-butylether kristallisiert. Es wurden 13,1 g der Titelverbindung mit einem Schmelzpunkt von 86 bis 90 °C erhalten.

### Beispiel 11:

### 3-{1-[2'-(tert.-Butylcarbonyloxymethoxycarbonyl)-4'-phenylbutyl]-cyclopentan-1-carbonylamino}-2,3,4,5-tetrahydro-2-oxo-1H-1-benzazepin-1-essigsäure-benzylester.

2 g 3-[1-(2'-Carboxy-4'-phenyl-butyl)-cyclopentan-1-carbonylamino]-2,3,4,5-tetrahydro-2-oxo-1H-1-benzazepin-1-essigsäurebenzylester (Herstellung siehe Beispiel 10) wurden unter Feuchtigkeitsausschluß in 20 ml trockenem Dichlormethan gelöst. Zu der Lösung wurden 0,46 ml Triethylamin und 0,1 g Dimethylaminopyridin gegeben. Dann wurde unter Eiskühlung eine Lösung von 0,5 g Pivalinsäurechlormethylester in 3 ml trockenem Dichlormethan zugetropft. Das Reaktionsgemisch wurde anschließend 2 Tage bei Raumtemperatur gerührt. Zur Aufarbeitung wurde das Reaktionsgemisch auf Wasser gegeben, die organische Phase abgetrennt, mit wäßriger Natriumbicarbonat-Lösung und anschließend mit Wasser gewaschen, über Natriumsulfat getrocknet und unter vermindertem Druck eingeengt. Das als Rückstand verbleibende Rohprodukt wurde durch Flash-Chromatographie an 150 g Kieselgel gereinigt, wobei als Elutionsmittel ein n-Hexan/Essigsäure-ethylester-Gemisch mit einer Zusammensetzung von zunächst 7:3 und dann 1:1 eingesetzt wurde. Es wurden 1,1 g reines 3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäurebenzylester als fester Schaum mit einem Schmelzbereich von 71 - 78 °C erhalten.

### Beispiel 12:

### 3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäure.

1,0 g 3-{1-[2'-(Pivaloyloxymethoxycarbonyl)-4'-phenyl-butyl]cyclopentan-1-carbonylamino}-2,3,4,5-tetrahydro-2-oxo-1H-1-benzazepin-1-essigsäure-benzylester (Herstellung siehe Beispiel 11) wurden in 100 ml Ethanol gelöst. Die Lösung wurde mit 0,5 g Palladium/Kohle-Katalysator (5 %-ig) versetzt. Dann wurde 3 Stunden mit einem Wasserstoffdruck von 5 bar hydriert. Zur Aufarbeitung wurde vom Katalysator abfiltriert und die filtrierte Lösung eingedampft. Der erhaltene Rückstand wurde bei 80 °C unter vermindertem Druck getrocknet. Es wurden 0,7 g der Titelverbindung als glasartiges Produkt erhalten.

IR-Spektrum (als KBr-Preßling): 3410 cm⁻¹, 1750 cm⁻¹, 1660 cm⁻¹

Nach den in den vorstehenden Beispielen beschriebenen Verfahren können auch die in der nachfolgenden Tabelle 1 aufgeführten Verbindungen der Formel I hergestellt werden.

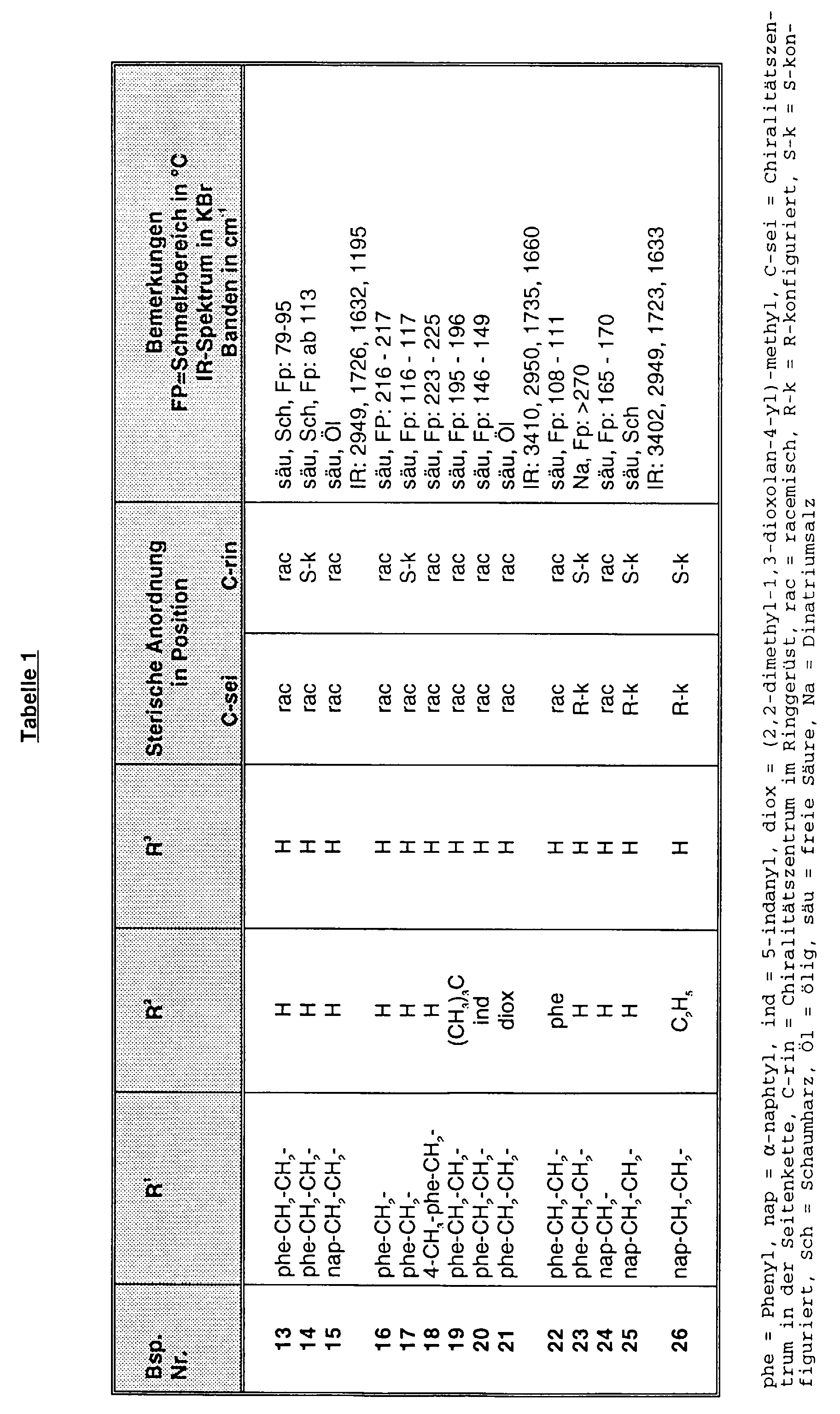

## Patentansprüche

1. Verwendung von Verbindungen der allgemeinen Formel I 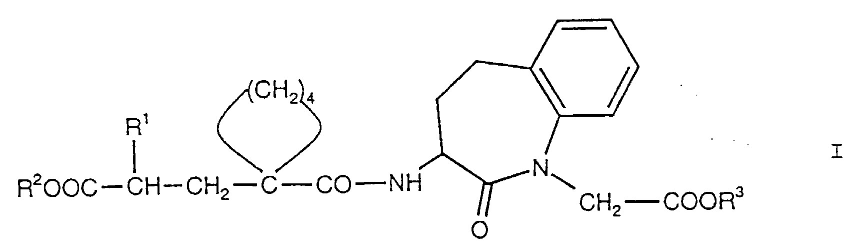 worin
R¹ für eine Phenyl-C1-C4-alkylgruppe, welche gegebenenfalls im Phenylring durch C1-C4-Alkyl, C1-C4-Alkoxy oder Halogen substituiert sein kann, oder für eine Naphthyl-C1-C4-alkylgruppe steht,
R² Wasserstoff oder eine einen biolabilen Ester bildende Gruppe bedeutet und
R³ Wasserstoff oder eine einen biolabilen Ester bildende Gruppe bedeutet,
und physiologisch verträglichen Salzen der Säuren der Formel I zur Herstellung von pharmazeutischen Zubereitungen zur Behandlung und/oder Prophylaxe von gastrointestinalen Durchblutungsstörungen.

2. Verwendung von Verbindungen gemäß Anspruch 1, worin R² und/oder R³ eine einen biolabilen Ester bildende Gruppe bedeuten.

3. Verwendung von Verbindungen nach einem der vorstehenden Ansprüche, worin die einen biolabilen Ester bildende Gruppe eine C1-C4-Alkylgruppe darstellt, eine gegebenenfalls im Phenylring durch C1-C4-Alkyl oder durch eine an 2 benachbarte Kohlenstoffatome gebundene niedere Alkylenkette substituierte Phenyl- oder Phenyl-C1-C4-alkylgruppe, insbesondere Phenyl, Benzyl oder Indanyl, darstellt, eine im Dioxolanring gegebenfalls durch C1-C4-Alkyl substituierte Dioxolanylmethylgruppe, insbesondere (2,2-Dimethyl-1,3-dioxolan-4-yl)methyl, darstellt, oder eine gegebenenfalls an der Oxymethylgruppe durch C1-C4-Alkyl substituierte C₂-C₆-Alkanoyloxymethylgruppe darstellt.

4. Verwendung von Verbindungen nach einem der vorstehenden Ansprüche dadurch gekennzeichnet, daß R² eine einen biolabilen Ester bildende Gruppe bedeutet und R³ Wasserstoff ist.

5. Verwendung von Verbindungen gemäß Anspruch 4, dadurch gekennzeichnet, daß (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure oder deren physiologisch verträgliche Salze eingesetzt werden.

## Claims

1. Use of compounds of the general formula I 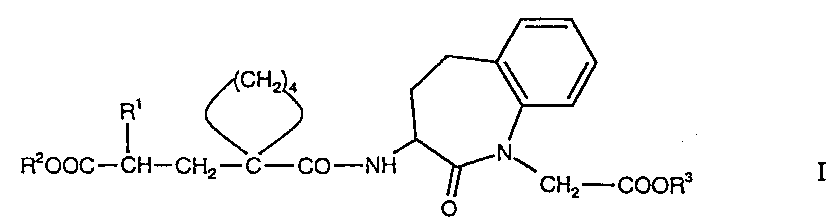 in which
R¹ represents a phenyl-C1-C4-alkyl group which can optionally be substituted in the phenyl ring by C1-C4-alkyl, C1-C4-alkoxy or halogen, or represents a naphthyl-C1-C4-alkyl group,
R² denotes hydrogen or a group forming a biolabile ester, and
R³ denotes hydrogen or a group forming a biolabile ester,
and physiologically tolerated salts of the acids of the formula I for preparing pharmaceutical compositions for the treatment and/or prophylaxis of gastrointestinal blood circulation disturbances.

2. Use of compounds according to Claim 1, in which R² and/or R³ denote(s) a group forming a biolabile ester.

3. Use of compounds according to one of the preceding claims, in which the group forming a biolabile ester represents a C1-C4-alkyl group; a phenyl or phenyl-C1-C4-alkyl group which is optionally substituted in the phenyl ring by C1-C4-alkyl or by a lower alkylene chain which is bonded to 2 adjacent carbon atoms, in particular phenyl, benzyl or indanyl; a dioxolanyl methyl group which is optionally substituted in the dioxolane ring by C1-C4-alkyl, in particular (2,2,-dimethyl-1,3-dioxolan-4-yl)methyl; or a C₂-C₆-alkanoyloxymethyl group which is optionally substituted on the oxymethyl group by C1-C4-alkyl.

4. Use of compounds according to one of the preceding claims, characterized in that R² denotes a group forming a biolabile ester and R³ is hydrogen.

5. Use of compounds according to Claim 4, characterized in that (3S,2'R)-3-{1-[2'-(ethoxycarbonyl)-4'-phenylbutyl]cyclopentane-1-carbonylamino)-2,3,4,5-tetrahydro-2-oxo-1H-1-benzazepine-1-acetic acid, or its physiologically tolerated salts, is/are employed.

## Revendications

1. Utilisation de composés de formule générale I 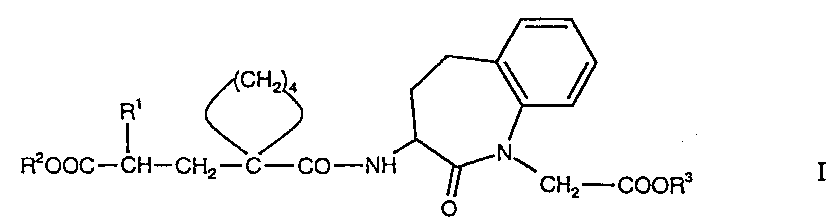 dans laquelle
R¹ représente un groupe alkyle en C₁ à C₄-phényle, lequel peut être éventuellement substitué dans le noyau phényle par un alkyle en C₁ à C₄, un alcoxy en C₁ à C₄ ou un halogène, ou représente un groupe alkyle en C₁ à C₄-naphtyle,
R² est un hydrogène ou un groupe formant un ester biolabile, et
R³ est un hydrogène ou un groupe formant un ester biolabile,
et des sels physiologiquement acceptables des acides de formule I pour l'élaboration de préparations pharmaceutiques destinées au traitement et/ou à la prophylaxie de troubles de l'irrigation sanguine gastro-intestinale.

2. Utilisation de composés selon la revendication 1, dans lesquels R² et/ou R³ représente(nt) un groupe formant un ester biolabile.

3. Utilisation de composés selon l'une quelconque des revendications précédentes, dans lesquels le groupe formant un ester biolabile est un groupe alkyle en C₁ à C₄, un groupe phényle ou un groupe alkyle en C₁ à C₄-phényle éventuellement substitué dans le noyau phényle par un groupe alkyle en C₁ à C₄ ou par une chaîne alkylène inférieur reliée à deux atomes de carbone voisins, en particulier un groupe phényle, benzyle ou indanyle, un groupe dioxolanylméthyle éventuellement substitué dans le noyau dioxolane par un alkyle en C₁ à C₄, en particulier un (2,2-diméthyl-1,3-dioxolan-4-yl)méthyle, ou un groupe alcanoyle en C₂ à C₆-oxyméthyle éventuellement substitué au niveau du groupe oxyméthyle par un alkyle en C₁ à C₄.

4. Utilisation de composés selon l'une quelconque des revendications précédentes, caractérisée en ce que R² est un groupe formant un ester biolabile et R³ est un atome d'hydrogène.

5. Utilisation de composés selon la revendication 4, caractérisée en ce que l'on utilise de l'acide (3S,2'R)-3-{1-[2'-(éthoxycarbonyl)-4'-phényl-butyl]-cyclopentan-1-carbonylamino}-2,3,4,5- tétrahydro-2-oxo- 1H-1-benzazépino-1-acétique ou ses sels physiologiquement acceptables.
